# EUROPEAN PATENT APPLICATION

(11) **EP 0 995 738 A1**
(43) Date of publication of application: **26.04.2000**
(21) Application number: 99120221.9
(22) Date of filing: 11.10.1999
(51) Int. Cl.: C07C 67/08, C07C 69/54

(54) **Process for the preparation of (meth)acrylate esters and polyester (meth)acrylates**

(30) Priority: 19.10.1998 US 174412
(71) Applicant: UCB, S.A., 1070 Bruxelles (BE)
(72) Inventor: Tweedy, Harrel E., Acworth, Georgia 30101 (US)

(57) **Abstract**

The present invention relates to a process for preparing acrylate esters, methacrylate esters, polyester acrylates and/or polyester methacrylates by reacting acrylic and/or methacrylic acid with a monohydroxy containing compound and/or a polyhydroxy containing compound in a reaction vessel, in the presence of a catalyst, a polymerization inhibitor and dense phase carbon dioxide. The dense phase carbon dioxide is in either a sub-critical, near-critical, critical or supercritical physical state. Advantages of using dense phase carbon dioxide as the solvent include the preparation of (meth)acrylate esters and polyester (meth)acrylates in an environmentally friendly manner by reducing or eliminating the need for hydrocarbon solvents, reducing or eliminating the problems associated with handling hydrocarbon solvents, simplifying recovery of products, and recovery and recycle of reactants as compared to the conventional esterification synthesis. In addition, the products produce by the process of this invention can have improved color and purity.

## Description

The present invention relates to a process for preparing acrylate esters, methacrylate esters, polyester acrylates or polyester methacrylates by reacting acrylic or methacrylic acid with a monohydroxy containing compound or a polyhydroxy containing compound in a reaction vessel in the presence of a catalyst and a polymerization inhibitor.

Conventionally, methacrylate esters and acrylate esters have been prepared via a direct esterification reaction by reacting methacrylic acid or acrylic acid, usually in excess, with an alcohol or a polyol in the presence of an azeotroping solvent for water removal, typically a hydrocarbon solvent, an esterification catalyst and a polymerization inhibitor. Limited examples are also known where the synthesis can also occur in the absence of a hydrocarbon solvent, for example, European Patent Application 0 202 610 by BASF Corporation and European Patent Application 0 376 090 by Henkel Corporation. In addition, (meth)acrylate esters have been prepared using transesterification reaction conditions as described in U.S patents 5,498,751 and 5,554,785.

Dense phase carbon dioxide has been used in the synthesis of organic compounds. For example, U.S. Patent 5,523,420 to Lowack et al. discloses preparing alpha-tocopherol using an acid catalyst and carbon dioxide as a solvent.

U.S. Patent 5,403,739 to Ikushima et al., discloses a method of esterifying (S)-citronellol using lipase in supercritical carbon dioxide. The product is prepared by reacting a fatty acid with citronellol using a lipase as the catalyst. The temperature and pressure of the carbon dioxide are adjusted to yield the (S)-ester from the racemic citronellol compound.

Kamat et al., *Biotechnology and Bioengineering, 40,* 158-166 (1992) discloses the transesterification of (meth)acrylates in supercritical solvents with an enzyme catalyst. This article suggests that carbon dioxide is not a good solvent for this transesterification since carbon dioxide acts as an inhibitor of the enzyme catalyst in the transesterification of acrylates.

Supercritical carbon dioxide has been used as a reaction medium for preparing polypeptides as is shown in U.S. Patents 5,001,224 and 5,241,048 to Barstow et al.

In addition, supercritical carbon dioxide has been used in the polymerization of monomers to form polymers. For example, U.S. Patent 5,478,910 to Russel et al., discloses the production of polyesters using supercritical fluids, such as carbon dioxide, in the presence of an enzyme catalyst.

U.S. Patent 5,312,882 to DeSimone et al., discloses the heterogeneous polymerization of monomers in the presence carbon dioxide. Included in the monomers are (meth)acrylate monomers, such as alkyl (meth)acrylates. The polymerization takes place in the presence of a surfactant that solubilizes the monomer in the carbon dioxide. This patent does not disclose the preparation of (meth)acrylates or polyester (meth)acrylates in carbon dioxide.

Romack, et al, *Macromolecules*, 28, 912-915 (1995), discloses the homopolymerization of acrylic acid in supercritical carbon dioxide.

WO 96/39456 discloses the preparation of polyester polymers in the presence of dense phase carbon dioxide as the polymerization medium. Typically, the monomers used in the processes of both patents are monomers which are capable of forming polyester polymers and include hydroxy carboxylic acid monomers and a mixture of diacid monomers and diol monomers.

Dense phase carbon dioxide has also been used in many other processes, such as the decaffeination of coffee and tea, extraction of cholesterol from egg yolks and the extraction of hops. However, dense phase carbon dioxide has not been used as a reaction aid in the direct esterification reaction of a mono-ol and/or polyol with acrylic acid and/or methacrylic acid.

It has been discovered that dense phase carbon dioxide can be used in a direct esterification synthesis of low molecular weight acrylate esters, methacrylate esters, polyester acrylates and/or polyester methacrylates. Synthesis of acrylate esters, methacrylate esters, polyester acrylates and/or polyester methacrylates in the presence of dense phase carbon dioxide provides a method to prepare the methacrylate and acrylate products in an environmentally friendly manner by reducing, or eliminating, the need for hydrocarbon solvents and the problems associated with handling hydrocarbon solvents. Significant reduction or elimination in handling of hydrocarbon solvents contribute to the positive environmental benefits from the process of this invention.

It has been further discovered that the products recovered from the process of this invention, in many instances, have improved color as compared to conventional hydrocarbon solvent and neat (solventless) processes.

Further, in the process of this invention, there is a potential reduction in processing costs and a reduction in the waste of raw materials used to prepare the commercially important acrylates and methacrylates. The reduction in processing cost is due to the ability to isolate the finished product in high purity and with fewer processing steps than in conventional esterification processes. In addition, due to the solubility characteristics of many of the reactants and reaction product(s) in the dense phase carbon dioxide, in many instances, the unconverted reactants are easily recovered from the dense phase carbon dioxide and/or are easily separated from the methacrylate and/or acrylate products that are present in the dense phase carbon dioxide. This allows the unconverted reactants to be recycled back into the reaction vessel for further reaction, hence reducing the cost of raw materials and/or allows the reaction products of the process to be separated in relatively high purity for future use.

The present invention relates to a process for preparing an acrylate ester, a methacrylate ester, a polyester acrylate, a polyester methacrylate or mixtures thereof comprising reacting acrylic acid, methacrylic acid or a mixture of acrylic acid and methacrylic acid with at least one monohydroxy containing compound and/or at least one polyhydroxy containing compound in a reaction vessel, in the presence of a catalyst, a polymerization inhibitor and dense phase carbon dioxide.

The term "dense phase carbon dioxide" encompasses carbon dioxide in a sub-critical physical state, a near-critical physical state, a critical physical state and carbon dioxide in the supercritical physical state.

In the present invention, the term "(meth)acrylic acid" encompasses both acrylic acid and methacrylic acid. Likewise, the term "(meth)acrylate" encompasses both acrylates and methacrylates and "polyester (meth)acrylate" encompasses both polyester acrylates and polyester methacrylates.

The term "polyester (meth)acrylate" is defined as a (meth)acrylate derived from a polyol that may have one or more (meth)acrylate functional groups present in the final product.

The process of this invention can be used to produce both monoesters and polyester compounds and/or mixtures thereof Monoesters are produced by reacting monohydroxy containing compounds with (meth)acrylic acid. The monohydroxy containing compounds which can be used in the invention include, but are not limited, to C₁₋₁₈ aliphatic monohydroxy compounds which are linear or branched, such as methanol, ethanol, butanol, propanol, isopropanol, octanol, decanol or mixtures of these compounds, and cycloaliphatic monohydroxy compounds such as cyclohexanol or hydroxyethylcyclohexanol. In addition, these monohydroxy containing compounds may be reacted with various amounts of ethylene oxide and/or propylene oxide to produce ethoxylated and/or propoxylated modified monohydroxy containing compounds. In addition, aromatic monohydroxy compounds such as phenol, phenoxyethanol or reaction products of phenol and various amounts of ethylene oxide and/or propylene oxide, can also be used.

Polyester (meth)acrylates are produced by reacting a polyhydroxy containing compound with (meth)acrylic acid. The polyhydroxy containing compounds, also called polyols, which can be used in the invention include compounds which contain more than one hydroxy group and are also compatible with the reaction conditions. Generally, the polyols which can be used in this invention have 2 to 10 hydroxy groups, preferably have 2 to 6 hydroxy groups and have 2 to about 36 carbon atoms. These polyols include branched and linear aliphatic polyols, cycloaliphatic polyols, aromatic polyols, polyether polyols and polyester polyols. Examples of aliphatic polyols include, but are not limited to, diols such as ethylene glycol, propylene glycol, butanediol, hexanediol, neopentyl glycol, tripropylene glycol, triethylene glycol, tetraethylene glycol and dimethylolpropane; triols, such as glycerine, trimethylolpropane, and trimethylolethane; tetra-ols, such as pentaerythritol and di-trimethylolpropane and hexa-ols such as dipentaerythritol. Examples of cycloaliphatic polyols include, but are not limited to, dimethylolcyclohexane. In addition, the aliphatic and cycloaliphatic polyols may be reacted with various amounts of ethylene oxide and/or propylene oxide to produce ethoxylated and/or propoxylated polyols. Examples of the ethoxylated and/or propoxylated polyols include, but are not limited to, ethoxylated hexanediol, propoxylated hexanediol, ethoxylated trimethylolpropane, propoxylated trimethylolpropane, ethoxylated glycerine, propoxylated glycerine, ethoxylated pentaerythritol, and propoxylated pentaerythritol. Examples of aromatic polyols include, but are not limited to, bisphenol A, bisphenol F, and reaction products of bisphenol A or bisphenol F with various amounts of ethylene oxide and/or propylene oxide. The polyether polyols which can be used in the present invention include both aromatic and aliphatic polyethers. The aliphatic groups of the polyether polyols can be linear, branched or cyclic. Examples of polyether polyols include, but are not limited to, di- and tri-glycols, such as diethylene glycol and triethylene glycol, polyethylene glycol, polypropylene glycol, polybutylene glycol and mixed polyethers, such as poly(propylene-ethylene)glycol. Polyester polyols are polyols which have ester linkages. For example, an excess of polyol is reacted with a diacid or the anhydride of the diacid using organic synthesis known to those skilled in the art to produce a low molecular weight compound having about 1 to about 6 ester linkages and reactive hydroxy groups. Any of the aforementioned polyols or mixtures of the aforementioned polyols can be used to prepare the polyester polyols. Examples of the diacids which can be used to produce polyester polyol include, but are not limited to, aliphatic diacids such as succinic acid and adipic acid, and aromatic diacids such as phthalic acid and terephthalic acid. In addition, cycloaliphatic diacid precursor anhydrides such as succinic anhydride can also be used to prepare the polyester polyols. Finally, the polyether and polyester polyols can also reacted with various amounts of ethylene oxide and/or propylene oxide to produce ethoxylated and/or propoxylated polyols.

In the process of the present invention, the monohydroxy containing compound can be used alone or in combination with other monohydroxy containing compounds and/or polyhydroxy containing compounds. Likewise, the polyhydroxy containing compounds can be used alone or in combination with other polyhydroxy containing compounds and/or monohydroxy containing compounds. In addition, the acrylic acid and methacrylic acid can be use alone or in combination with each other. Of course, when a mixture of the hydroxy containing reactants is used in the process of this invention, mixed ester products are obtained. The same is true when a mixture of acrylic and methacrylic acids is used, the product will be a mixed ester product. When a specific compound is desired as the product, however, the process should be performed with a single hydroxy containing reactant and either acrylic or methacrylic acid accordingly, depending on the desired product.

Additionally, other functional groups can be present on the hydroxy functional compound provided the other functional groups are compatible with the general esterification process and carbon dioxide.

In the process of the present invention, the dense phase carbon dioxide can act as a solvent for some of the reactants. Other reactants, however, are not soluble or are only slightly soluble in the dense phase carbon dioxide. For these non-soluble or slightly soluble reactants, the dense phase carbon dioxide still functions to facilitate the reaction by creating a process state where there is a molecular dilution of the reactants. It is theorized, although not being bound by theory, that the dense phase carbon dioxide functions to mediate reaction processes such as heat transfer and molecular interactions as well as perform some other solvent-like functions which typically occur when solvents are actually present. Likewise, the dense phase carbon dioxide can contribute these properties in a positive manner to otherwise solventless or neat reactions.

In the process of the present invention, the goal is to produce (meth)acrylate functional products. Therefore, it is desirable to functionalize at least about 5.0% of the hydroxy functionality in the starting hydroxy functional compound with (meth)acrylic acid. Preferably, about 50% to about 100% of the hydroxy functional groups should be functionalized.

To ensure that at least 5.0% of the hydroxy functional groups are functionalized, the amount of the (meth)acrylic acid reactant should vary between about 0.05 moles and about 2.0 moles of the (meth)acrylic acid per mole of hydroxy functional groups. Preferably, there should be between about 0.5 moles and about 2.0 moles of (meth)acrylic acid reactant per mole of hydroxy functional group.

In the process of this invention, it is desirable to use a catalyst to promote the esterification reaction. Conventional esterification or transesterification catalysts, which are solid, liquid or a combination of a solid and a liquid and which exhibit at least some esterification catalysis activity at temperatures below 150°C, can be used in the process of this invention. Typical catalysts include sulfuric acid, sulfonic acids, ion exchange resins, mineral acids, acidic clays, certain metal compounds and supported catalysts. The sulfonic acid catalysts include, but are not limited to, para-toluene sulfonic acid, benzenesulfonic acid methanesulfonic acid and derivatives thereof The ion exchange resins are preferably those resins having sulfonic acid functionality such as AMBERLYST A-15 available from Rohm & Haas Company, DOWEX-type available from Aldrich Chemical Company or other resins which also contain sulfonic acid functionality. Mineral acids include, but are not limited, to sulfuric acid. In addition, supported catalyst can be used, such as an acid treated K-10 Montmorillonite, available from Aldrich Chemical Company. Generally, the non-supported catalyst is used in an amount in the range of about 0.5 wt.% to about 10 wt.% based on the weight of the reactants in the reaction mixture, not including the carbon dioxide, and will vary depending on the effectiveness of the catalyst. Using a solid catalyst or a supported catalyst, such as ion exchange resins, the effective concentration of catalytic sites present is the important feature to consider not the specific mass of the solid or supported catalyst. It is noted, however, that the reaction vessel should contain a sufficient amount of the catalyst to ensure that the reaction will proceed at a reasonable rate. Agitation of the contents of the reaction vessel will help promote contact of the reactants with the catalyst. The catalyst can also be heterogeneous and present in the reaction vessel as a solid which is either dispersed in the reaction mixture or is concentrated in a zone in the reaction vessel through which the reactants must pass.

In the case of heterogeneous solid catalyst, such as ion exchange resins, resin beads of various dimensions may also be used wherein the reactants pass through a bed or reactor zone containing the resin beads and come into contact with the catalytic active sites. The reactants may come into contact with these active sites by the physical and solubility transport properties of the dense phase carbon dioxide.
Polymerization inhibitors may also be used in the process of the present invention. These inhibitors, sometimes also called stabilizers, help retard or prevent the polymerization of the reactants during the reaction process and prevent or minimize the further reactions or degradation of the products produced by the esterification process of the present invention. The inhibitors are typically present at the start of the reaction process, typically being present in the (meth)acrylic acid reactant, at least to some extent, and/or the inhibitors can be additionally added before, during, and/or after the reaction process has taken place. Conventional polymerization inhibitors which can be used in the present invention include quinone-type inhibitors, such as hydroquinone, methylether of hydroquinone and various other substituted quinones known in the art, certain copper containing materials, such as copper(I) oxide, and copper carbonates, other known stabilizers such as phenothiazine, methylene blue and many of those stabilizers mentioned in U.S. Patents 4,053,504 to Rosenkranz et al., U.S. Patent 3,899,740 to Broussard et al., and U.S. Patent 4,059,721 to Rosenkranz et al., each incorporated by reference. These stabilizers can generally be used in combination with air (oxygen), which has inhibitive properties of its own, in combination with one another, or they can be used alone. The effectiveness of the inhibitors may be impacted, either positively or negatively, by the conditions, such as the presence of oxygen or temperature, etc., which are present before, during and after the reaction. The amount of the polymerization inhibitor added to the reaction mixture is any amount conventionally used in the art. Typically, the polymerization inhibitor is added in an amount up to about 30,000 ppm based on the weight of the (meth)acrylic acid in the reaction mixture. In the process of the present invention, the polymerization inhibitors are preferably present in an amount up to 10,000 ppm, preferably in an amount of 10-10,000 ppm based on the weight of the (meth)acrylic acid in the reaction mixture. The inhibitor is preferably selected from the group consisting of hydroquinone, methylether of hydroquinone, phenothiazine, methylene blue, copper(I)oxide, copper carbonate, and a combination thereof. Additionally, the presence of some oxygen in the carbon dioxide stream can be beneficial.

As is well known, all gases have a critical temperature and pressure. The critical temperature is the temperature above which the gas cannot be liquefied by an increase in the pressure, and the critical pressure is the pressure above which the liquid can not be converted to a gas by an increase in the temperature. For carbon dioxide, the critical temperature is 31.1°C. and the critical pressure is 73.8 bar. The term "supercritical carbon dioxide", as used herein, refers to carbon dioxide at a temperature greater than 31.1 °C. and a pressure greater than 73.8 bar. Again, the term dense phase carbon dioxide refers to carbon dioxide having at least partially liquid-like properties and may be either sub-critical, critical, or supercritical physical state. The range of temperature and pressure which will produce dense phase carbon dioxide is well known to those skilled in the art.

In the present invention, it is preferred that the reaction takes place in the presence of dense phase carbon dioxide in its supercritical physical form. The reaction proceeds more readily at temperatures above the critical temperature and the solubility of the reactants and products is more readily tunable to achieve optimum results when the pressure is near, at or above the critical pressure. In such case, the temperature in the reactor should be in the range between 31.1°C and about 140°C and the pressure should be in the range between about 70 bar and about 400 bar.

Dense phase carbon dioxide in its supercritical state has a number of properties making it a very desirable reaction solvent. It is inert under a wide variety of organic reaction conditions. It has a high diffusivity and is readily able to penetrate solid phase supports. By varying the temperature and/or pressure, the solubility parameters can be varied to selectively dissolve, un-dissolve (precipitate) or phase separate various reactants and reaction products. Using dense phase carbon dioxide in the esterification reaction of this invention also minimizes problems in recovery of reaction products because the carbon dioxide is removed simply by reducing pressure to conditions where the dense phase carbon dioxide becomes gaseous in form, thereby avoiding the need for washings, distillations, and other laborious and costly separation processes typically practiced in organic synthesis to remove spent reagents, side products and other reaction solvents.

It has also been discovered that preparing (meth)acrylates and/or polyester (meth)acrylates in the presence of dense phase carbon dioxide, as opposed to conventional hydrocarbon solvents or neat reactions, many times improves the color of the products isolated from the reaction process. It is believed, although not wishing to be bound by theory, that the reduction in color is a result of the milder reaction conditions, i.e. lower temperatures and shorter reaction times, dilution of the reactants as compared to neat reactions, the absence of hydrocarbon solvent impurities in solvent reactions and/or product isolation conditions employed with the process of this invention.

Although it is preferred that dense phase carbon dioxide is employed without any additional solvents, small amounts of other non-reactive solvents compatible with (meth)acrylate esters and dense phase carbon dioxide may be used as a co-solvent. Co-solvents can be useful to enhance solubility of reactants which are difficult to dissolve in the dense phase carbon dioxide. In selecting a co-solvent, the co-solvent should have very different solubility properties in dense phase carbon dioxide from the products being isolated at a given temperature and pressure condition. This will make it relatively easy to separate the co-solvent from the desired product by controlling the pressure in the let-down step processing of the dense phase carbon dioxide. The co-solvent may be used alone or in combination with other co-solvents. The total amount of the co-solvents should be limited to no more than about 5% by volume, based on the volume of the carbon dioxide. Further, it is necessary that the co-solvent(s) is not reactive with the reactants and is stable in the dense phase carbon dioxide. Preferably, the co-solvent is a hydrocarbon compound. Examples of acceptable co-solvents include cyclohexane, hexane, and other similar compounds. It is preferred that additional co-solvents are not used in the present invention since the introduction of solvents introduces the various problems associated with solvent handling, such as environmental problems, removal from the reaction mixture, and/or final product purification.

Using carbon dioxide in the absence of a co-solvent, in the process of the present invention, has the advantages of improved efficiency, minimal waste disposal, lower material cost, etc. In addition, the products of the present invention produced without a co-solvent solvent may have improved quality in that there can be a lower color, fewer by-products, etc., than when the same products are produced by conventional thermal esterification reactions.

The reactor used in the present invention should be a pressure capable vessel which has no stirring means or, more preferably, which is capable of agitating the contents of the reactor. A "static" reaction process is one where the reactants are placed in the reactor and the carbon dioxide is charged to the reactor. The reactor's internal pressure is increased to the desired reaction pressure to achieve carbon dioxide in a dense phase. This is achieved by various methods known in the art including, but not limited to, direct charging of the carbon dioxide under pressure, charging the carbon dioxide as a gas and increasing pressure and temperature in the reaction vessel such as to increase the density of the carbon dioxide or a combination of both. The temperature is also increased by methods known in the art, such as externally heating the reaction vessel, internally heating the reaction vessel or through heating resulting from the compression of the carbon dioxide.

Generally, the reaction mixture is held at an elevated temperature for a given period of time to achieve at least 5 % conversion of the hydroxyl groups to esters. Preferably, the reaction mixture is held at an elevated temperature for a sufficient amount of time to achieve at least 50% conversion of the hydroxyl groups, and more preferably, with water removal techniques and/or reaction product removal techniques being employed, to achieve at least 85% or more conversion of the hydroxyl functional groups to (meth)acrylates. Heating of the reaction vessel can be accomplished via an external or internal heating source or can be heated via the compression of the carbon dioxide in the reaction vessel. Typically, the reaction temperature is in the range of about room temperature (about 23°C) to about 140°C, preferably in the range of about 60°C to 120°C, more preferably in the range of 70°C to 100°C.

Typically, at least one external or internal heating source may be needed in addition to the heating of the reactor by increasing the pressure of the contents of the reactor. Once the reactor is heated and pressurized, the reactor is held at, or near this temperature and pressure for a specific period of time. The desired temperature and pressure values necessary for optimal reaction will vary depending on specific reactants being used, primarily due to their differing solubility in dense phase carbon dioxide and other reactivity properties. While good solubility of the reaction in dense phase carbon dioxide is preferred, it is not required for the process of this invention to be successful. Dense phase carbon dioxide can act either as a transport, dilution and/or solubility agent for the reactants. Solubility of the product (meth)acrylates and/or polyester (meth)acrylates is also preferred, but is again not absolutely required. The time period, in which the reactor is held at a desired temperature and pressure, will vary depending on the reaction conditions and the desired yield of the product and product composition.

A stirred reactor is typically equipped with a stirring means. This stirring means serves to agitate the contents of the reactor. Stirring can be typically accomplished by using a magnetic stir bar and external rotating magnetic stirrer which will agitate the contents of the reactor. Other stirring or agitation means, known to those skilled in the art, can also be used. An inert support, which supports the reactants and/or catalyst, may or may not be present. If an inert support is present, the agitation mechanism must be suitable for stirring particulates of the support. Alternatively, the reactor contents may be agitated or stirred by passing dense phase carbon dioxide through the reaction mixture via appropriate introduction methods known in the art including a sparge tube arrangement. In this arrangement, the dense phase carbon dioxide is flowing through the reactor, while maintaining approximately the same temperature and pressure. In addition, the reactor can also be equipped with a movable gasket which will allow pressure of the reaction vessel to be adjusted accordingly, a viewing window for visual access to the reaction chamber, and/or other equipment variations known in the art.

The reaction mixture, containing the hydroxy functional compound(s) and (meth)acrylic acid, as well as the catalyst and polymerization inhibitor(s), may also be absorbed on an inert solid support. Examples of inert solid supports include, but are not limited to, glass wool, diatomaceous earth, hydromatrix or other solid materials. The reaction mixture in combination with the solid adsorbent support are loaded into the reaction vessel and the dense phase carbon dioxide is brought into contact with the reaction mixture and support material by any of the methods practiced by those skilled in the art.

The reactants, catalyst and stabilizers may be added initially, before pressurization of the carbon dioxide, added intermittently in a semi-continuous process or continuously fed to the reaction vessel in conjunction with the carbon dioxide. The reaction mixture used in the process of the present invention can be either premised prior to addition or introduction into the reaction vessel, simultaneously fed to the reaction vessel as separate streams, or various components can be combined in a single stream while other components are fed in different streams to the reaction vessel. In addition, the process can be carried out in a batch process, semi continuous batch, a continuous process or a combination of one or more of these processes.

Typically, in a batch process, the hydroxy functional compound and (meth)acrylic acid, catalyst and polymerization inhibitors are added to the reaction vessel, prior to the introduction of the carbon dioxide into the reaction vessel. The carbon dioxide can be added to the reaction vessel either in a gaseous form or a liquid form. If the carbon dioxide is added to the reaction vessel in a gaseous form, the pressure and/or temperature must be adjusted to convert the gaseous carbon dioxide to dense phase carbon dioxide. Once the carbon dioxide is charged to the reaction vessel, the contents of the reaction vessel are heated, if necessary. Generally, some heat is supplied to the reaction vessel if the compression of the carbon dioxide does not sufficiently heat the reaction vessel to a temperature which will effect the reaction of the reactants. Further, if liquid carbon dioxide is added to the reaction vessel at a temperature below a temperature which will effect the reaction, heat must be applied to the reaction vessel to effect the reaction. Again, heat may be applied to the reaction vessel by means of an external heating means or an internal heating means to maintain the temperature at desired levels. Once the reaction has reached the desired level of conversion, the pressure of the reaction vessel is reduced by venting the carbon dioxide from the reaction vessel. The reaction products are then recovered from the reactor and/or the vented carbon dioxide, depending on the solubility properties of the reaction products in the carbon dioxide.

Generally, in a semi-continuous batch process, the initial charge of the reaction mixture proceeds in a manner similar to the batch process. The reaction is allowed to proceed for a period of time. At that point, some of the mixture in the reaction vessel is removed by venting some or all of the carbon dioxide from the reaction vessel. Carbon dioxide, either fresh or recycled, may again be added to the reaction vessel. Optionally, additional reactants may also be added to the reaction vessel. This series of process steps can be repeated at least one additional time. Preferably, the series of steps is repeated several times to achieve the desired level of conversion of the starting material. It is preferred that the pressure and temperature of the contents in the reaction vessel be returned to and/or maintained at approximately the same pressure and temperature. The pressure can be maintained at or about the same pressure by adding additional carbon dioxide to the reaction vessel followed by compressing the contents of the reaction vessel if necessary. Alternatively, the pressure in the reaction vessel can be maintained by compressing the carbon dioxide remaining in the reaction mixture after the venting step. Once the reaction has reached the desired level of conversion, the contents of the reaction vessel are vented to recover the desired (meth)acrylate products.

In a continuous process, the reactants, carbon dioxide and possibly the catalyst and/or polymerization inhibitors are continuously supplied to the reaction vessel. Generally, the catalyst is located in the reaction vessel as a catalyst zone or catalyst bed. The reactants will pass though a catalyst zone or catalyst bed in the reactor causing the reaction to proceed. Unconverted reactants, esterification products, by-products and carbon dioxide are continuously removed from the reaction vessel. The product can be recovered from the unconverted reactants, by-products and carbon dioxide using conventional separation techniques known to those skilled in the art.

The carbon dioxide, used in the process of this invention, can be vented to the atmosphere. Conversely, the carbon dioxide can be recovered in moderate to high purity reasonably easily and can be recycled to the process. It is beneficial to recover the carbon dioxide since this will reduce production cost and it is fairly easy to recover the carbon dioxide. The carbon dioxide can be recovered from the reaction vessel by reducing the pressure in the reactor or venting the pressure through various collection vessels at lower pressures as is known in the art for supercritical fluid extraction technology.

Some residual amounts of reactants and/or products may be soluble in the venting carbon dioxide, hence these reactants and/or products will be removed from the reactor and will initially remain in solution with the carbon dioxide, depending on the pressure and temperature conditions under which the release is being controlled. Therefore, it is beneficial to either recycle the carbon dioxide back into the reactor without removing the dissolved components or with further treatment of the carbon dioxide to recover the dissolved components, such as unconverted reactants, products or by-products, from the carbon dioxide. It is beneficial to recycle, recover and/or trap the carbon dioxide from an environmental stand point since products and/or reactants may be lost and released to the environment if the carbon dioxide is simply vented to the atmosphere. By recycling the carbon dioxide back into the reactor or recovering the unconverted reactants or products, the operational cost associated with the purchase of the raw materials can be reduced. In addition, by removing the product from the carbon dioxide released from the reaction vessel the yield of the desired product can be enhanced, which will in turn, help reduce the production cost of producing the desired products. The carbon dioxide can be treated by methods, such as further pressure/temperature reductions, as are known in the art of supercritical fluid extraction technology, to cause the dissolved materials to separate from the carbon dioxide, by adsorptive methods for the raw materials and/or products, as are known in the art, or by other purification processes known to those skilled in the art. Recycling of the carbon dioxide is particularly advantageous when a continuous flow of dense phase carbon dioxide is used in the process of this invention, since carbon dioxide is typically a solvent for some of the reactants and/or products, as is stated above. Using a continuous flow of the dense phase carbon dioxide may cause these reactants and products to be removed from the reactor along with the carbon dioxide flowing out of the reactor prior to achieving the desired extent of esterification. Therefore recovery of the carbon dioxide and further isolation of or recycle of the materials as dissolved components in the carbon dioxide is desirable to maximize raw material efficiency and product productivity and recovery.

It is desirable to remove water, products and/or other generated by-products from the reaction mixture to achieve the optimum degree of desired esterification. This is due to the fact that the reaction taking place is an equilibrium reaction, hence removing water, products and/or by-products will cause the equilibrium to shift towards complete conversion of hydroxyl groups to corresponding (meth)acrylates. However, it is not critical to the present invention to remove water, products and/or the by-products to successfully produce (meth)acrylate esters and/or polyester (meth)acrylates in the presence of dense phase carbon dioxide. The reaction will proceed to a moderate to substantial degree of completion without removing the water, products and/or by-products from the reaction vessel. This degree of completion will depend upon various parameters, including reactant stoichiometries.

Any conventional water removing technique, known to those skilled in the art, and also compatible with dense phase carbon dioxide pressurization can be used. Applicable water removal, product and/or by-product removal techniques during pressure venting include, but are not limited to, sweeping the vapor area above the liquid reactants, sparging the reaction mixture, or doing both with an inert gas or gas mixture which is compatible with carbon dioxide and the stability of the reaction mixture and the raw materials. Carbon dioxide can be used alone as a sparge during pressure venting to remove water, products and/or by products from the reaction vessel or other gases can be incorporated in the carbon dioxide mixture. Air can also be used as a component in the carbon dioxide and is particularly suitable when quinone-type inhibitors or other oxygen-activated inhibitors are present, since it will additionally assist with reactant and product stabilization. Preferably, the generated water, products and/or by-products may be removed from the reaction zone by continuous carbon dioxide venting or stepwise venting of the reaction vessel either while maintaining reaction conditions in the reaction vessel or in a cyclical venting/repressurization cycle with carbon dioxide.

Once the water, products and/or by-products are removed from the reaction vessel, they can be trapped by conventional means such as using cold traps. One convenient way to remove water from the pressurized reaction zone is to use a continuous flow of dense phase carbon dioxide into the reaction zone and concurrent depressurization flow from the reactor zone. The pressure of the carbon dioxide flowing from the reactor is reduced through pressure release valving and the water which has dissolved in the dense carbon dioxide will be separated due to reduced solubility as the carbon dioxide pressure is lowered and becomes gaseous as well as simple solubility saturation of water in the gas. Other drying methods for the carbon dioxide stream known in the art, such as molecular sieves and membranes are also possible water removal options. A cold trap, with or without additional water adsorption methods including physical adsorption, can also be used. Absorptive agents such as DRIERITE available from Aldrich Chemical Company and molecular sieves, are effective agents which can be used to remove the water from the carbon dioxide gas stream prior to recycle of the carbon dioxide. Likewise, removed esterified product can be recovered, fractionated and purified using techniques known in the art of supercritical fluid extraction.

In the process of this invention, the carbon dioxide pressure in the reaction vessel can be reduced and additional reactants and/or carbon dioxide can be charged to the reaction vessel as in a semi-continuous batch processing mode. The pressure of the freshly added carbon dioxide can again be increased, as necessary, to give dense phase carbon dioxide. The addition of the additional reactants to the reaction vessel serves to further react any initial charge of remaining reactants in the reaction vessel which may have not reacted in the first pressurization as well as achieve reaction of the newly added reactants.

As is described above, carbon dioxide is released from the reaction vessel to allow recovery of the products of the process and for other reasons as mentioned above. The pressure of the carbon dioxide is reduced through appropriate valving which is properly configured to minimize plugging due to the temperature reduction which occurs upon decompression (Joule-Thompson effect) to allow recovery of the dense phase carbon dioxide-dissolved materials, including reactants, products, co-solvents, etc. Recovery of the these material can be accomplished by techniques known in the art for supercritical fluid extraction in an appropriate collection vessel and may be in the form of liquids, solids or a combination of both depending upon the specific properties of the dissolved products and the temperatures and pressures present in the collection vessel.

The final products produced by the process of this invention may also be recovered and isolated from the reaction mixture and reaction process by using conventional techniques known in the art. Included in these techniques are water washing, solvent washing, neutralization, solvent removal and distillative techniques as well as other processes known in the art of esterification and general organic chemistry. In addition, if the products of the process of this invention contain some water from the esterification reaction and the products are soluble in carbon dioxide, the products can be removed from the water by further extracting the aqueous products with dense phase carbon dioxide. The carbon dioxide soluble products are solubilized by the dense phase carbon dioxide into the extract phase. This extract phase, containing the dense phase carbon dioxide and the carbon dioxide soluble reaction products, is subject to a reduction in pressure, converting the dense phase carbon dioxide to gaseous carbon dioxide. When the carbon dioxide changes to the gaseous phase, the products are recovered.

The invention is a process for preparing an acrylate ester, a methacrylate ester, a polyester acrylate, a polyester methacrylate or mixtures thereof comprising reacting acrylic acid, methacrylic acid or a mixture of acrylic acid and methacrylic acid with a hydroxy containing compound selected from the group consisting of a monohydroxy containing compound, a polyhydroxy containing compound, a mixture of monohydroxy containing compounds, a mixture of polyhydroxy containing compounds and a mixture of monohydroxy containing compounds and polyhydroxy containing compounds in a reaction vessel, in the presence of a catalyst, a polymerization inhibitor and dense phase carbon dioxide.

In the process of the invention, the acrylic acid, methacrylic acid or mixture of acrylic acid and methacrylic acid, the hydroxy containing compound, the catalyst and the polymerization inhibitor are first placed in the reaction vessel, then the reaction vessel is charged with carbon dioxide gas and/or carbon dioxide liquid and pressurized. The reaction vessel is heated to effect the reaction, wherein the heat is provided by a source selected from the group consisting of an external heating source, an internal heating source, compression of the carbon dioxide in the reaction vessel and a combination thereof

The process of the invention can comprise the steps of
(a) charging the reaction vessel with a reaction mixture comprising the acrylic acid, methacrylic acid or an mixture of acrylic acid and methacrylic acid, the hydroxy containing compound, the catalyst, and the polymerization inhibitor,
(b) charging the reaction vessel with the carbon dioxide gas or liquid carbon dioxide,
(c) increasing the pressure and optionally the temperature in the reaction vessel to convert the carbon dioxide gas to dense phase carbon dioxide, and
(d) heating the contents of the reaction vessel for a sufficient period of time to effect the reaction between the acrylic acid, methacrylic acid or mixture of acrylic and methacrylic acid and the hydroxy containing compound.
   The process of the invention can also comprise the step of (e) venting the reaction vessel after the reaction has proceeded. The process can also comprise the step (e) venting the reaction vessel during the reaction while maintaining the temperature and pressure of the reaction vessel by
   1) adding additional carbon dioxide gas or liquid and compressing the contents in the reaction vessel to convert the carbon dioxide to dense phase carbon dioxide and/or
   2) compressing the contents remaining in the reaction vessel after said venting of the reaction vessel.

   The process of the invention comprises the additional steps of
(e) reducing the pressure in the reaction vessel by venting the reaction vessel,
(f) adding additional acrylic acid, methacrylic acid and a mixture of acrylic acid and methacrylic acid and, optionally, adding additional hydroxy containing compound to the reaction vessel,
(g) optionally adding additional carbon dioxide to the reaction vessel
(h) increasing the pressure in the reaction vessel, and
(i) heating the contents of the reactor for a sufficient period of time to effect the reaction between the acrylic acid and/or methacrylic acid and the hydroxy containing compound.

In the process, step (e) can be repeated at least one time additional time to increase the yield of the acrylate ester, methacrylate ester, polyester acrylate and/or polyester methacrylate. In the process, steps (e), (f), (g), (h) and (i) can be repeated at least one time addition time to increase the yield of the acrylate ester, methacrylate ester, polyester acrylate and/or polyester methacrylate. In the process the step (e) can be repeated a sufficient number of times to achieve at least 85% conversion of the starting hydroxy groups in the hydroxy containing compound. In the process steps (e), (f), (g), (h) and (i) can be repeated a sufficient number of times to achieve at least 85% conversion of the starting hydroxy groups in the hydroxy containing compound.

The process can also comprise a step of venting the carbon dioxide from the reaction vessel after the reaction has proceeded to recover the acrylate ester, a methacrylate ester, a polyester acrylate and/or a polyester methacrylate reaction product from the reaction vessel and/or the vented carbon dioxide.

In the process the reaction is conducted in the presence of dense phase carbon dioxide and in the absence of a co-solvent.

The reaction can be conducted in the further presence of an inert co-solvent. The co-solvent is present in an amount up to 5% based on the volume of the dense phase carbon dioxide.

The process of the invention can also comprise the step of venting the carbon dioxide from the reaction vessel, the vented carbon dioxide contains some dissolved reactants, products and/or by-products, and carbon dioxide is recycled back into the reaction vessel, with or without removing essentially all of the dissolved component from the carbon dioxide.

The process wherein the reaction mixture is adsorbed on an inert solid support, can comprise the steps of
1) absorbing the acrylic acid, methacrylic acid or mixture of acrylic acid and methacrylic acid, the hydroxy containing compound, catalyst, and polymerization inhibitor onto an inert solid support to form supported reactants,
2) loading the supported reactants into the reaction vessel,
3) charging the reaction vessel with carbon dioxide gas and/or carbon dioxide liquid,
4) increasing the pressure and optionally the temperature in the reaction vessel to convert the charged carbon dioxide to dense phase carbon dioxide, and
5) holding the contents of the reaction at about a constant temperature and about a constant pressure under static conditions to effect the reaction.

In another embodiment of the process of the invention, the acrylic acid, methacrylic acid or mixture of acrylic acid and methacrylic acid, the hydroxy containing compound, catalyst, and polymerization inhibitor are place into the reaction vessel in the absence of an inert support.

The process of the invention can also comprise an additional process step of removing water, reaction product and/or other volatile components generated by the reaction from the reaction vessel. The water, reaction product and/or other volatile components are trapped after removal from the reaction vessel. The water, reaction product and/or other volatile or soluble components are removed by sparging and/or bleeding the carbon dioxide from the reaction vessel.

In another embodiment of the invention, the process comprises the additional steps of
(a) reducing the pressure in the reaction vessel,
(b) adding additional acrylic acid and/or methacrylic acid and optionally adding additional hydroxy containing compound to the reaction vessel,
(c) optionally adding additional carbon dioxide to the reaction vessel,
(d) increasing the pressure and optionally the temperature in the reaction vessel, and
(e) allowing reaction to proceed.

In the process, the acrylic acid, methacrylic acid or mixture of acrylic acid and methacrylic acid and the hydroxy containing compound are simultaneously fed to the reactor. In another embodiment, the acrylic acid, methacrylic acid or mixture of acrylic acid and methacrylic acid and the hydroxy containing compound are premixed prior to addition to the reactor.

In the process of the invention, the monohydroxy containing compounds are preferably selected from the group consisting of C₁₋₁₈ linear or branched aliphatic monohydroxy compounds, cycloaliphatic monohydroxy compounds, aromatic monohydroxy compounds and ethoxylated and/or propoxylated derivative thereof; and the polyhydroxy compounds are preferably selected form the group consisting of polyols having about 2 to about 10 hydroxy groups and 2 to about 36 carbon atoms and ethoxylated and/or propoxylated derivatives thereof The monohydroxy containing compounds are preferably selected from the group consisting of methanol, ethanol, cyclohexanol, octanol, and decanol; and the polyhydroxy compounds are preferably selected from the group consisting of cyclohexanediol, butanediol, hexanediol, tripropylene glycol, tetraethylene glycol, trimethylolpropane, pentaerythritol, dipentaerythritol, di-trimethylolpropane, triethylene glycol, ethoxylated hexanediol, propoxylated hexanediol, ethoxylated trimethylolpropane, propoxylated trimethylolpropane, ethoxylated glycerine, propoxylated glycerine, ethoxylated pentaerythritol, and propoxylated pentaerythritol.

In the process of the invention, the catalyst is preferably selected from the group consisting of para-toluene sulfonic acid, methanesulfonic acid, benzenesulfonic acid, sulfonic acid ion-exchange resins, sulfuric acid, and acid clays.

In the process of the invention, either acrylic acid or methacrylic acid is reacted with either a single monohydroxy containing compound or a single polyhydroxy containing compound.

In the process of the invention, the prepared acrylate ester, methacylate ester, polyester acrylate, polyester methacrylate or mixtures thereof, contains water and is extracted with subcritical, critical or supercritical dense phase carbon dioxide. The carbon dioxide is converted from a dense state to a gaseous state by reducing the pressure.

The following examples are present to give those skilled in the art a better understanding of the claimed invention. The examples are in no way intended to limit the invention.

### EXAMPLES

### Example 1

2.82 gm of trimethylolpropane, 5.0 gm of acrylic acid, and 0.1 gm of phenothiazine were mixed together and adsorbed onto 3.47 gm of hydromatrix to form a reactant mixture. A 100 ml cylindrical supercritical fluid extraction/reaction cell, configured for carbon dioxide entry and exit at different ends of the cell, was loaded in layers from the bottom up in the following order: 5.6 gm of hydromatrix / 11.3 gm of the reactant mixture / 2.3 gm of hydromatrix / 2.0 gm of AMBERLYST A-15 ion exchange resin / 1.0 gm of hydromatrix. Carbon dioxide was charged to the cell and the cell was pressurized to 105 bars at 70°C. The contents of the cell were held under this static condition for fifteen minutes. After which time, a carbon dioxide extraction flow was slowly and continually bleed from the cell over about a 4 hour period. During this 4 hour period, the cell pressure was held at 105 bar for 1 hour, after which the pressure was raised to 125-130 bar with the temperature being held in the 73-76°C range during the next 3 hours. The carbon dioxide extraction was continuous, amounting to a total volume of about 256 liters of carbon dioxide gas (measured as a gas at ambient temperature and pressure using a gas flow meter) over the 4 hour period. Sample aliquots were collected using cold trapping in dry ice chilled U-shaped collection tubes throughout the carbon dioxide bleed time. After the 4 hour period, the extraction/reaction cell was then fully depressurized and the contents of the depressurization process were isolated using cold trapping. 5.6 gm of total weight loss (sum of all steps) from the reaction cell occurred. However, only 3.8 gm of total product were actually collected due to incomplete trapping of volatiles, such as acrylic acid. No indication of gel formation was observed. Gas Chromatography analysis for trimethylolpropane and its acrylated derivatives in the final 1.8 gm of extract which was collected during the extraction process indicated (approximately): 12.7 area % trimethylolpropane triacrylate, 49.5 area % trimethylolpropane diacrylate, 24% trimethylolpropane monoacrylate, 3 area % trimethylolpropane, and the remainder longer retention time materials and other residual products. Additionally, a non-measured amount of acrylic acid was also present. The total cell weight loss equals about 70% of theory, based upon initial charge of trimethylolpropane and acrylic acid reactants. The contents of the support matrix were extracted with acetone. Analysis of an acetone extraction of the remaining support matrix material by Gas Chromatography indicated (approximately): 92 area % unreacted trimethylolpropane in the remaining 30% of reactant material (acrylic acid, trimethylolpropane, and residual trimethylolpropane acrylates) not extracted from the reaction cell by the venting or bleeding of carbon dioxide from the cell.

### Example 2

2.8 gm of trimethylolpropane, 9.2 gm of acrylic acid, and 0.2 gm of phenothiazine were mixed together. To this mixture, 10.0 gm of AMBERLYST A-15 ion exchange resin catalyst was added. Finally, 10.0 gm of hydromatrix was added and the mixture was mixed to form a flowable moist powder. The mixture was loaded into a 100-ml cylindrical supercritical fluid extraction/reaction cell configured for carbon dioxide entry and exit at different ends of the cell. Carbon dioxide was charged to the cell and pressurized to about 110 bar at 80°C. The mixture was held under this static condition for about four hours. Following this hold time, controlled venting of the carbon dioxide from the cell was conducted to extract the contents of the cell. During this venting process, the total reaction cell pressure was maintained at about 110 bar through addition and compression of fresh carbon dioxide to the cell. The venting carbon dioxide was passed through a U-tube collection container submerged in dry ice. Product recovery from the carbon dioxide occurs as the carbon dioxide pressure returns to ambient conditions. The amount of carbon dioxide vented by this extraction step was measured to be about 165 liters (at ambient temperature and pressure) with a gas flow meter. The extraction/reaction cell was then fully depressurized without additional carbon dioxide being charged to the extraction/reaction cell. Additional products from the depressurization process were isolated using the same cold trap as described above. Gas Chromatography was used to analyze the contents of the collection tube. Approximately 75 wt. % of the collected fraction was acrylic acid, based upon acid value determination. The other approximately 0.75 gm contents of the collection tube (excluding the acrylic acid present) were found by Gas Chromatographic analysis for acrylated and nonacrylated trimethylolpropane to be (approximately): 72 area % trimethylolpropane triacrylate, 20 area % trimethylolpropane diacrylate and the remainder higher molecular weight material or other residual products. The recovered acrylated and non-acrylated trimethylolpropane species yield equals about 13% of theory based upon initial charge of trimethylolpropane reactant. The remaining reaction cell contents were removed from the cell, mixed with an additional 5.0 gm acrylic acid, re-loaded into the reaction cell, and re-pressurized with carbon dioxide and held at 110 bar and 80°C an additional 2 hours. After which, another extraction of the reaction cell was conducted and the contents of this second extraction step were isolated in a similar manner to the first step. During the second extraction, a total extraction flow of approximately 170 liters of carbon dioxide gas (measured at ambient temperature and pressure) was removed from cell while the extraction/reaction cell is maintained at pressure and temperature by addition and compression of fresh carbon dioxide. Approximately 50 wt. % of the collected fraction was acrylic acid, based upon acid value determination. The other approximately 2.84 gm contents of the collection tube (excluding the acrylic acid present) were found by Gas Chromatographic analysis for acrylated and non-acrylated trimethylolpropane species to be (approximately): 74 area % trimethylolpropane triacrylate, 10.6 area % trimethylolpropane diacrylate, and the remainder higher molecular weight materials and other residual products. The recovered acrylated and non-acrylated trimethylolpropane species yield equals approximately 48% of theory based on initial charge of trimethylolpropane. Following this extraction, the extraction/reaction cell was fully depressurized without adding additional carbon dioxide to the cell. Gas Chromatography analysis of an acetone extraction of the remaining support matrix, which is calculated to be about 40% of theory acrylated and non-acrylated trimethylolpropane species based on the initial of trimethylolpropane, indicated (approximately): 27 area % trimethylolpropane triacrylate, 21 area % trimethylolpropane diacrylate, 11 area % trimethylolpropane monoacrylate, 5 area % trimethylolpropane polyol, and 23 area % higher molecular weight product composition in addition to other residual products. Total (calculated) degree of trimethylolpropane hydroxyl esterification for the reaction sequences is about 87%.

### Example 3

2.8 gm of trimethylolpropane, 8.9 gm of acrylic acid, and 0.1 gm of phenothiazine were mixed together. To this mixture, 10.1 gm of AMBERLYST A-15 ion exchange resin catalyst was added. Finally, 10.1 gm of hydromatrix was added to the mixture and the mixture was mixed to yield a flowable moist powder. A 100 ml cylindrical supercritical fluid extraction/reaction cell, configured for carbon dioxide entry and exit at different ends of the cell, was loaded from the bottom up with 5.1 gm of hydromatrix followed by 32.0 gm of the above mixture. Carbon dioxide was charged to the cell and the cell pressurized to about 100 bars at 77°C. The mixture was held under this static condition for two hours. After which, the pressure was increased to 120 bar. The mixture was held an additional 2 hours at 120 bar and at a temperature range of 74-76°C. The extraction/reaction cell was then vented to allow a slow extraction by carbon dioxide while maintaining the reaction vessel pressure in the 125-130 bar range. The extraction was continued for approximately 3 hours, using a total of approximately 225 liters of carbon dioxide (measured as a gas at ambient temperature and pressure using a gas flow meter). Aliquots of the extraction stream were isolated by trapping in several dry ice chilled section of U-shaped collection tubes (tubes 1-2).

In tube #1, a total of 3.5 gm of liquid was isolated from approximately 83 liters of carbon dioxide gas (measured as a gas at ambient temperature and pressure) and was analyzed by Gas Chromatography for acrylated and non-acrylated trimethylolpropane species. It was found to contain (approximately): 20 area % of trimethylolpropane diacrylate, 73 area % trimethylolpropane triacrylate, and 3+ area % longer retention time materials and other residual products. Acid value determination indicated this aliquot to also contain about 72 wt. % acrylic acid.

In tube #2, a total of 2.0 gm of extracted product was isolated from approximately 88 liters of carbon dioxide (measured as a gas at ambient temperature and pressure) extraction flow and was analyzed by Gas Chromatography for acrylated and non-acrylated trimethylolpropane species. It contained (approximately): 13 area % of trimethylolpropane diacrylate, 79 area % trimethylolpropane triacrylate and 4+ area % longer retention time materials and other residual products. Acid value determinations indicated this aliquot to also contain about 18 wt. % acrylic acid.

The extraction cell was fully decompressed without carbon dioxide replacement. Following this full decompression of the reaction cell replacement, the remaining hydromatrix sample was extracted with acetone and analyzed by Gas Chromatography for acrylated and non-acrylated trimethylolpropane species. It contained (approximately): 4.9 area % trimethylolpropane, 13.4 area % trimethylolpropane monoacrylate, 18.3 area % trimethylolpropane diacrylate, 35.6 area % trimethylolpropane triacrylate, 16+ area % higher molecular weight and other residual products. Acid value determination on this aliquot indicated about 18 wt % free acrylic acid.

### Example 4

2.8 gm of trimethylolpropane, 9.0 gm of acrylic acid, and 0.13 gm of phenothiazine were mixed together. To this mixture, 9.9 gm AMBERLYST A-15 ion exchange resin catalyst was added. Finally, 7.6 gm of hydromatrix was added and the entire mixture was mixed to yield a flowable moist powder. Following a preliminary addition of 4.6 gm of hydromatrix into the 100-ml cylindrical supercritical fluid extraction/reaction cell, which is configured for carbon dioxide entry and exit at different ends of the cell reaction cell, the flowable moist powder was loaded into the cell. Carbon dioxide was charged and pressurized to about 110-115 bars at 73-75°C. The mixture was held under these static conditions for approximately four hours. The extraction/reaction cell was then depressurized without any further carbon dioxide addition or compression to minimize any extraction of the cell contents. The extraction/reaction cell's hydromatrix was extracted with acetone and the resulting extract was analyzed for reaction products. Gas Chromatography area % analysis for acrylated and non-acrylated trimethylolpropane species indicated (approximately) 1.4 area % trimethylolpropane, 6.3 area % trimethylolpropane monoacrylate, 31.5 area % trimethylolpropane diacrylate, 46.5 area % trimethylolpropane triacrylate, and 9+ area % other residuals.

### Example 5 (control, no catalyst)

A mixture of 2.8 gm of trimethylolpropane, 8.4 gm of acrylic acid, and 0.2 gm of phenothiazine and 16.9 gm hydromatrix was prepared. This mixture was loaded into a 100-ml cylindrical supercritical fluid extraction/reaction cell, which is configured for carbon dioxide entry and exit at different ends of the cell. Carbon dioxide was charged and pressurized to about 125 bars at about 73-75°C. The mixture was held at these conditions for four hours. The extraction/reaction cell was then extracted and approximately 50 liters of carbon dioxide were evolved while the extraction/reaction cell temperature and pressure were maintained by addition and compression of additional carbon dioxide. The extraction product was collected in a dry ice chilled U-tube through which the carbon dioxide gas flowed. Gas Chromatographic analysis for acrylated and non-acrylated trimethylolpropane species indicated only starting material trimethylolpropane was present. No significant amount of acrylated polyol product was found by Gas Chromatography analysis of the collected extractant or from the extraction/reaction cell contents following full decompression and extraction of the hydromatrix.

### Example 6

3.0 gm of 1,6-hexanediol, 8.0 gm of acrylic acid, and 0.3 gm of phenothiazine were mixed together. To this mixture, 10.2 gm of AMBERLYST A-15 ion exchange resin catalyst was added. Finally, 10.0 gm of hydromatrix was added and the mixture was mixed to yield a flowable moist powder. The mixture was loaded into a 100-ml cylindrical supercritical fluid extraction/reaction cell, which is configured for carbon dioxide entry and exit at different ends of the cell. Carbon dioxide was charged and pressurized to about 95 bars at 72°C. The mixture was held static under this condition for about 2.5 hours. Extraction of the extraction/reaction cell by controlled venting of the carbon dioxide through chilled collection tubes, while maintaining the extraction/reaction cell temperature and pressure through addition and compression of fresh carbon dioxide, yielded the following aliquots:
- Tube 1:: (0.3 gm collected at pressure of about 100 bar for this collection). Analysis by Gas Chromatography for acrylated and nonacrylated 1,6-hexanediol species showed (approximately): 6.6 area % 1,6-hexanediol monoacrylate, 90.2 area % 1,6-hexanediol diacrylate, and 2+ area % higher retention time material and other product residues.
- Tube 2:: (0.9 gm collected at a pressure of about 110 bar). Analysis by Gas Chromatography for acrylated and nonacrylated 1,6-hexanediol species showed (approximately) 3.6 area % 1,6-hexanediol monoacrylate, 93.3 area % 1,6-hexanediol diacrylate, and 2+ area % higher retention time material and reaction residue.
- Tube 3:: (1.0 gm collected at pressure of about 130 bar): Analysis by Gas Chromatography for acrylated and non-acrylated 1,6-hexanediol species showed (approximately) 2.1 area % 1,6-hexanediol monoacrylate, 95.3 area % 1,6-hexanediol diacrylate, and 2+ area % higher retention time material and other product residue.

Then the extraction/reaction cell was fully decompressed without replacement of carbon dioxide. Following the decompression, an acetone extraction of the hydromatrix was performed and the extractant solution was isolated. Analysis of the extractant solution by Gas Chromatography for acrylated and non-acrylated 1,6-hexanediol species showed (approximately) 19.5 area % 1,6-hexanediol monoacrylate, 70 area % 1,6-hexanediol diacrylate, and 8+ area % higher retention time material and other product residues.

### Example 7

A mixture consisting of 5.0 gm of hydromatrix, 0.1 gm of phenothiazine, and 7.5 gm of acrylic acid was prepared (mixture #1). A second mixture of 10.0 gm of tripropylene glycol, 16.2 gm of AMBERLYST A-15 ion exchange resin, and 0.5 gm of hydromatrix was also prepared (mixture #2). A 100 ml cylindrical supercritical fluid extraction/reaction cell which is configured for carbon dioxide entry and exit at different ends of the cell was loaded from the bottom up in the order: 3.3 gm hydromatrix / mixture #1 / 1.9 gm hydromatrix / mixture #2 / 4.6 gm hydromatrix. Carbon dioxide was charged and pressurized to about 150 bars at 80°C. A slow continual bleed of carbon dioxide through the system was maintained throughout a 4 hour period while maintaining temperature at approximately 80°C during the entire 4 hour period. The pressure was maintained at 150 bar for the first 2.5 hours of the 4 hour period and 175 bar for the final 1.5 hours of the 4 hour period through the addition of and the compression of a fresh carbon dioxide charge to the cell. Samples were collected in various dry ice chilled U-shaped collection tubes at various times during the 4 hour period. The extraction/reaction cell was then allowed to be fully depressurized. The remaining extraction/reaction cell contents were extracted with acetone and this extractant analyzed. Samples were recovered as follows.
- Sample 1:: 0.77 gm collected after 22 min. reaction time in 5 liters carbon dioxide gas (as measured at ambient conditions) at 150 bar and 80°C. Analysis by Gas Chromatography for acrylated and non-acrylated tripropylene glycol species showed (approximately): 87.6 area % tripropylene glycol, 10.9 area % tripropylene glycol monoacrylate, 0 area % tripropylene glycol diacrylate, and 1 area % higher retention time components along with some other product residues.
- Sample 2:: 1.54 gm collected after 77 min. total reaction time in 20 liters carbon dioxide gas (as measured at ambient conditions) at 150 bar and 80-85°C. Analysis by Gas Chromatography for acrylated and non-acrylated tripropylene glycol species showed (approximately): 49.6 area % tripropylene glycol, 45.5 area % tripropylene glycol monoacrylate, 2.1 area % tripropylene glycol diacrylate, 0.1 area % higher retention time components as well as other residuals.
- Sample 3:: 1.07 gm collected after 130 min. total reaction time in 15 liters carbon dioxide gas as measured at ambient conditions) at 150 bar and 81°C. Analysis by Gas Chromatography for acrylated and non-acrylated tripropylene glycol species showed (approximately) 18 area % tripropylene glycol, 66.3 area % tripropylene glycol monoacrylate, 11.8 area % tripropylene glycol diacrylate, 0.3 area % higher retention time components as well as other residuals.
- Sample 4:: 3.99 gm collected after 180 min total reaction time in 35 liters of carbon dioxide gas (as measured at ambient conditions) at 150 - 175 bar and about 80°C. Analysis by Gas Chromatography for acrylated and non-acrylated tripropylene glycol species showed (approximately): 9.5 area % tripropylene glycol, 64.4 area % tripropylene glycol monoacrylate, 18.5 area % tripropylene glycol diacrylate, 1.0 area % higher retention time components along with other residual products.
- Sample 5:: 3.49 gm collected after 245 minutes total reaction time in 50 liters carbon dioxide gas (as measured at ambient conditions) at 175 - 180 bar and about 80°C. Analysis by Gas Chromatography for acrylated and non-acrylated tripropylene glycol species showed (approximately): 4.5 area % tripropylene glycol, 53.4 area % tripropylene glycol monoacrylate, 28.0 area % tripropylene glycol diacrylate, 1.9 area % higher retention time components along with other residual products.
- Sample 6:: An acetone-extracted hydromatrix from fully depressurized reaction cell after 295 minutes of lapsed time. Analysis by Gas Chromatography for acrylated and non-acrylated tripropylene glycol species showed (approximately) 34.3 area % tripropylene glycol, 38.6 area % tripropylene glycol monoacrylate, 9.4 area % tripropylene glycol diacrylate, and 3.3 area % higher retention time components and other residual products.

### Example 8

15.35 gm of tripropylene glycol, 0.5 gm of phenothiazine, 25.0 AMBERLYST A-15 ion exchange resin, 23.06 gm acrylic acid, and 25 gm of hydromatrix were thoroughly mixed. 38.9 gm of this mixture was loaded on top of 5.4 gm of hydromatrix in a cylindrical 100-ml. supercritical fluid extraction/reaction cell, which is configured for carbon dioxide entry and exit at different ends of the cell. The cell was pressurized to about 150 bar and held at 75°C for 3 hours at which time depressurization was begun without further addition of carbon dioxide or additional compression. Depressurization required about 1.5 hours. Less than 0.3 gm of material was collected in a dry ice cooled collection tube through which the carbon dioxide was vented. The remaining extraction/reaction cell's hydromatrix was extracted with toluene. Both samples were analyzed by Gas Chromatography for acrylated and non-acrylated tripropylene glycol species which indicated (approximately):

For the Extractant of carbon dioxide: 10.3 area % tripropylene glycol, 70.5 area % tripropylene glycol monoacrylate, 13.3 area % tripropylene glycol diacrylate, 2.3 area % higher retention time components and other residual products.

For the extracted hydromatrix excluding acrylic acid contribution: 3.2 area % tripropylene glycol, 65.1 % tripropylene glycol monoacrylate, 22.1 area % tripropylene glycol diacrylate, 4.2 area % higher retention time components and other residual products.

An identical amount (38.9 gm) of the noted reaction mixture was placed into a 100 ml round bottom flask. 50 gm of toluene was added to cover the mixture and the flask was held in a water bath at 75°C without agitation for 4.5 hours. The mixture was then cooled, stirred and filtered. The resulting filtrate was analyzed by Gas Chromatography. Analysis by Gas Chromatography for acrylated and non-acrylated tripropylene glycol species showed (approximately): 15.4 area % tripropylene glycol, 67.4 area % tripropylene glycol monoacrylate, 9.5 area % tripropylene glycol diacrylate, and 2.9 area % higher retention time components along with other residue products.

### Example 9

A mixture of 3.38 gm of trimethylolpropane, 10.66 gm of acrylic acid, and 0.16 gm of phenothiazine, 0.44 gm of methanesulfonic acid (70% in water) were loaded into a 60 ml variable volume view cell equipped with a magnetic stir bar, a sampling valve, and a movable gasket to allow pressurization. Carbon dioxide (17.8 gm) was charged to the vessel from a transfer vessel and the system pressurized by decreasing the vessel volume by closing the gasket. This compression also heats up the reaction mixture in addition to increasing the pressure. The vessel was then placed over a magnetic stirrer and the cell contents were agitated via a stir bar inside the cell. The reaction was held in the range of 5000 to 5200 psi and 73-83°C for 3 hours. During this time, samples were removed periodically through the sampling valve by expansion of the carbon dioxide directly into a sample vial. Analysis by Gas Chromatography for acrylated and non-acrylated trimethylolpropane species showed the following
- Sample 1:: Collected at 1.2 hours elapsed time indicated (approximately) 3.8 area % trimethylolpropane, 29.3 area % of trimethylolpropane monoacrylate, 45.3 area % trimethylolpropane diacrylate area 14.1 area % trimethylolpropane triacrylate, and 7.5 area % longer retention time materials.
- Sample 2:: Collected at 3.5 hours elapsed time indicated (approximately) 2.0 area % trimethylolpropane, 21.8 area % of trimethylolpropane monoacrylate, 47.9 area % trimethylolpropane diacrylate, 20.7 area % trimethylolpropane triacrylate, and approximately 7.6 area % longer retention time materials and other product residues.

## Claims

1. A process for preparing an acrylate ester, a methacrylate ester, a polyester acrylate, a polyester methacrylate or mixtures thereof comprising reacting acrylic acid, methacrylic acid or a mixture of acrylic acid and methacrylic acid with a hydroxy containing compound selected from the group consisting of a monohydroxy containing compound, a polyhydroxy containing compound, a mixture of monohydroxy containing compounds, a mixture of polyhydroxy containing compounds and a mixture of monohydroxy containing compounds and polyhydroxy containing compounds in a reaction vessel, in the presence of a catalyst, a polymerization inhibitor and dense phase carbon dioxide.

2. The process of claim 1, comprising the steps of
(a) charging the reaction vessel with a reaction mixture comprising the acrylic acid, methacrylic acid or an mixture of acrylic acid and methacrylic acid, the hydroxy containing compound, the catalyst, and the polymerization inhibitor,
(b) charging the reaction vessel with the carbon dioxide gas or liquid carbon dioxide,
(c) increasing the pressure and optionally the temperature in the reaction vessel to convert the carbon dioxide gas to dense phase carbon dioxide, and
(d) heating the contents of the reaction vessel for a sufficient period of time to effect the reaction between the acrylic acid, methacrylic acid or mixture of acrylic and methacrylic acid and the hydroxy containing compound.

3. The process of claim 2, further comprising the steps of
(e) venting the reaction vessel after the reaction has proceeded.

4. The process of claim 2, further comprising the step
(e) venting the reaction vessel during the reaction while maintaining the temperature and pressure of the reaction vessel by
1) adding additional carbon dioxide gas or liquid and compressing the contents in the reaction vessel to convert the carbon dioxide to dense phase carbon dioxide and/or
2) compressing the contents remaining in the reaction vessel after said venting of the reaction vessel.

5. The process of claim 2, comprising the additional steps of
(e) venting the reaction vessel in order to reduce the pressure in the reaction vessel,
(f) adding additional acrylic acid, methacrylic acid and a mixture of acrylic acid and methacrylic acid and, optionally, adding additional hydroxy containing compound to the reaction vessel,
(g) optionally adding additional carbon dioxide to the reaction vessel
(h) increasing the pressure in the reaction vessel, and
(i) heating the contents of the reactor for a sufficient period of time to effect the reaction between the acrylic acid and/or methacrylic acid and the hydroxy containing compound.

6. The process of claim 1, further comprising the step of venting the carbon dioxide from the reaction vessel, wherein the vented carbon dioxide contains some dissolved reactants, products and/or by-products, and carbon dioxide is recycled back into the reaction vessel, with or without removing essentially all of the dissolved component from the carbon dioxide.

7. The process of claim 1, wherein the acrylic acid, methacrylic acid or mixture of acrylic acid and methacrylic acid, the hydroxy containing compound, catalyst, and polymerization inhibitor are adsorbed on an inert solid support.

8. The process of claim 7, comprising the steps of
1) absorbing the acrylic acid, methacrylic acid or mixture of acrylic acid and methacrylic acid, the hydroxy containing compound, catalyst, and polymerization inhibitor onto an inert solid support to form supported reactants,
2) loading the supported reactants into the reaction vessel,
3) charging the reaction vessel with carbon dioxide gas and/or carbon dioxide liquid,
4) increasing the pressure and optionally the temperature in the reaction vessel to convert the charged carbon dioxide to dense phase carbon dioxide, and
5) holding the contents of the reaction at about a constant temperature and about a constant pressure under static conditions to effect the reaction.

9. The process of claim 1, further comprising an additional process step of removing water, reaction product and/or other volatile components generated by the reaction from the reaction vessel.

10. The process of claim 1, comprising the additional steps of
(a) reducing the pressure in the reaction vessel,
(b) adding additional acrylic acid and/or methacrylic acid and optionally adding additional hydroxy containing compound to the reaction vessel,
(c) optionally adding additional carbon dioxide to the reaction vessel,
(d) increasing the pressure and optionally the temperature in the reaction vessel, and
(e) allowing reaction to proceed.

11. The process of claim 1, wherein the monohydroxy containing compounds are selected from the group consisting of C₁₋₁₈ linear or branched aliphatic monohydroxy compounds, cycloaliphatic monohydroxy compounds, aromatic monohydroxy compounds and ethoxylated and/or propoxylated derivative thereof; and the polyhydroxy compounds are selected form the group consisting of polyols having about 2 to about 10 hydroxy groups and 2 to about 36 carbon atoms and ethoxylated and/or propoxylated derivatives thereof

12. The process of claim 1, wherein either acrylic acid or methacrylic acid is reacted with either a single monohydroxy containing compound or a single polyhydroxy containing compound.

13. The process of claim 1, wherein the prepared acrylate ester, methacylate ester, polyester acrylate, polyester methacrylate or mixtures thereof, contains water and is extracted with subcritical, critical or supercritical dense phase carbon dioxide.
